# EUROPEAN PATENT APPLICATION

(11) **EP 4 230 070 A1**
(43) Date of publication of application: **23.08.2023**
(21) Application number: 20965934.1
(22) Date of filing: 16.12.2020
(51) Int. Cl.: A24F 40/65

(54) **INHALATION DEVICE, TERMINAL DEVICE, AND PROGRAM**

(71) Applicant: Japan Tobacco Inc., Tokyo 105-6927 (JP)
(72) Inventor: AKAO, Takeshi, Tokyo 130-8603 (JP); NAGAHAMA, Toru, Tokyo 130-8603 (JP); AOYAMA, Tatsunari, Tokyo 130-8603 (JP)
(74) Representative: Hoffmann Eitle
(86) International application number: PCT/JP2020/047043
(87) International publication number: WO 2022/130546

(57) **Abstract**

[Problem] To provide a contrivance with which it is possible for the times at which information is obtained by an inhalation device to be calculated within a terminal device.

[Solution] This inhalation device comprises a generation unit that generates an aerosol using a base material, a storage unit that stores information, and a control unit that controls the action of the inhalation device, the control unit changing the value of a first counter each time a unit time period elapses, and the storage unit storing log information that includes the value of the first counter at a first timing at which a prescribed action relating to the inhalation device is performed.

## Description

### Technical Field

The present invention relates to an inhaler device, a terminal device, and a program.

### Background Art

Inhaler devices that generate a substance to be inhaled by the user, such as electronic cigarettes and nebulizers, are widely used. An inhaler device generates an aerosol with a flavor component, for example, using a substrate including an aerosol source for generating an aerosol and a flavor source for imparting a flavor component to the generated aerosol. A user can taste a flavor by inhaling the aerosol with the flavor component generated by the inhaler device.

During these years, it is being considered to provide various services by equipping an inhaler device with a communication function and allowing the inhaler device to communicate with a terminal device such as a smartphone. The following Patent Literature 1, for example, discloses a technique for displaying, using a terminal device, a use history of an inhaler device on the basis of information received from the inhaler device.

### Citation List

### Patent Literature

Patent Literature 1: WO 2020/006311 A

### Summary of Invention

### Technical Problem

In the above Patent Literature 1, however, no consideration is given to a fact that an inhaler device and a terminal device are not necessarily be connected to each other constantly. When an inhaler device and a terminal device are not connected to each other, it is difficult for the terminal device to immediately obtain information obtained by the inhaler device.

The present invention has been conceived in view of the above problem and aims to provide a mechanism in which a terminal device can calculate a time at which an inhaler device obtained information.

### Solution to Problem

In order to solve the above problem, according to an aspect of the present invention, there is provided an inhaler device including a generation unit that generates an aerosol using a substrate, a memory that stores information, and a controller that controls operation of the inhaler device. The controller changes a value of a first counter each time unit time has elapsed. The memory stores log information including the value of the first counter at a first timing, at which a certain operation relating to the inhaler device was performed.

The certain operation may include the generation unit starting to generate the aerosol.

The certain operation may include inhalation of the aerosol by the user

The inhaler device may further include a power supply that accumulates power and that supplies the power for operation of the inhaler device. The certain operation may include charging of the power supply.

If a first condition is satisfied, the inhaler device may permit the generation of the aerosol. The certain operation may include an operation that satisfies the first condition.

If a second condition is satisfied, the inhaler device may inhibit the generation of the aerosol. The certain operation may include an operation that satisfies the second condition.

The inhaler device may further include a communicator that communicates with another device. The communicator may transmit the log information stored in the memory at a second timing and then transmit the value of the first counter at the second timing.

The communicator may transmit information indicating the unit time.

The memory may remove the transmitted log information or overwrite the transmitted log information stored in a memory area with the new log information.

After the communicator receives an acknowledgment indicating that the log information was successfully received, the memory may remove the transmitted log information or overwrite the transmitted log information stored in a memory area with new log information.

The controller may start to change the value of the first counter when the inhaler device is activated for a first time.

The generation unit may heat an aerosol source as liquid guided from the substrate.

The generation unit may heat the substrate that contains an aerosol source and that is formed in a certain shape.

In order to solve the above problem, according to another aspect of the present invention, there is provided a terminal device includes a communicator that communicates with another device and a controller that performs processing based on information received by the communicator. The communicator receives log information including a value of a first counter at a first timing, at which a certain operation relating to an inhaler device, which generates an aerosol using a substrate, changes the value of the first counter each time unit time has elapsed, and stores the log information, was performed, and the value of the first counter at a second timing, at which the inhaler device transmitted the log information. The controller calculates a time corresponding to the first timing on a basis of the value of the first counter at the first timing, the value of the first counter at the second timing, and a time at which the communicator received the log information.

After the time corresponding to the first timing is calculated, the communicator may transmit an acknowledgment indicating that the log information was successfully received.

The terminal device may further include an outputter that outputs information. The outputter may output the time corresponding to the first timing.

The terminal device may further include a positional information obtainer that obtains positional information regarding the terminal device and a memory that stores the positional information obtained by the positional information obtainer while associating the positional information with a time at which the positional information was obtained. The outputter may output the positional information obtained at the time corresponding to the first timing.

The terminal device may communicate with the inhaler device through a relay device that communicates with the inhaler device. The relay device may relay, at the second timing, the log information received from the inhaler device to the relay device and also relay the value of the first counter at the second timing to the terminal device. The controller may calculate the time corresponding to the first timing on a basis of the value of the first counter at the first timing, the value of the first counter at the second timing, and the time at which the communicator received the log information.

The communicator may communicate with the inhaler device through a relay device that communicates with the inhaler device. The relay device may change a value of a second counter each time unit time has elapsed, receive the log information from the inhaler device at the second timing and then receives the value of the first counter at the second timing from the inhaler device, store the value of the second counter at the second timing along with the log information and the value of the first counter at the second timing, and transmit the log information at a third timing, at which the relay device communicates with the terminal device, and also transmits the value of the first counter at the second timing, the value of the second counter at the second timing, and the value of the second counter at the third timing. The controller may calculate the time corresponding to the first timing on a basis of the value of the first counter at the first timing, the value of the first counter at the second timing, the value of the second counter at the second timing, the value of the second counter at the third timing, and the time at which the communicator received the log information.

In order to solve the above problem, according to another aspect of the present invention, there is provided a program for causing a computer, which controls a terminal device including a communicator that communicates with another device, to calculate, when an inhaler device, which generates an aerosol using a substrate, changes a value of a first counter each time unit time has elapsed, and stores log information, receives the log information including the value of the first counter at a first timing, at which a certain operation relating to the inhaler device was performed, and the value of the first counter at a second timing, at which the inhaler device transmitted the log information, a time corresponding to the first timing on a basis of the value of the first counter at the first timing, the value of the first counter at the second timing, and a time at which the communicator received the log information.

### Advantageous Effects of Invention

As described above, according to the present invention, a mechanism in which a terminal device can calculate at a time at which an inhaler device obtained information is provided.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 is a diagram illustrating an example of the configuration of a system according to a first embodiment.
[Fig. 4] Fig. 4 is a diagram illustrating an example of a display screen for log information displayed by a terminal device according to the embodiment.
[Fig. 5] Fig. 5 is a sequence diagram illustrating an example of a procedure of a process performed by a system according to the embodiment.
[Fig. 6] Fig. 6 is a diagram illustrating an example of the configuration of a system according to a second embodiment.
[Fig. 7] Fig. 7 is a diagram illustrating a method for calculating a time corresponding to a first timing according to the embodiment.

### Description of Embodiments

A preferred embodiment of the present invention will be described in detail hereinafter with reference to the accompanying drawings. Components having substantially the same functional configuration will be given the same reference numerals herein and in the drawings, and redundant description thereof is omitted.

In addition, elements having substantially the same functional configuration might be given different alphabets after the same reference numeral herein and the in the drawings. For example, a plurality of elements having substantially the same functional configuration will be distinguished from each other like inhaler devices 100A and 100B as necessary. When a plurality of elements having substantially the same functional configuration need not be particularly distinguished from each other, however, only the same reference numeral will be given. When the inhaler devices 100A and 100B need not be particularly distinguished from each other, for example, the inhaler devices 100A and 100B will be simply referred to as inhaler devices 100.

### « 1. Configuration Example of Inhaler Device»

An inhaler device generates material to be inhaled by a user. In the example described below, the material generated by the inhaler device is an aerosol. Alternatively, the material generated by the inhaler device may be gas.

### (1) First configuration example

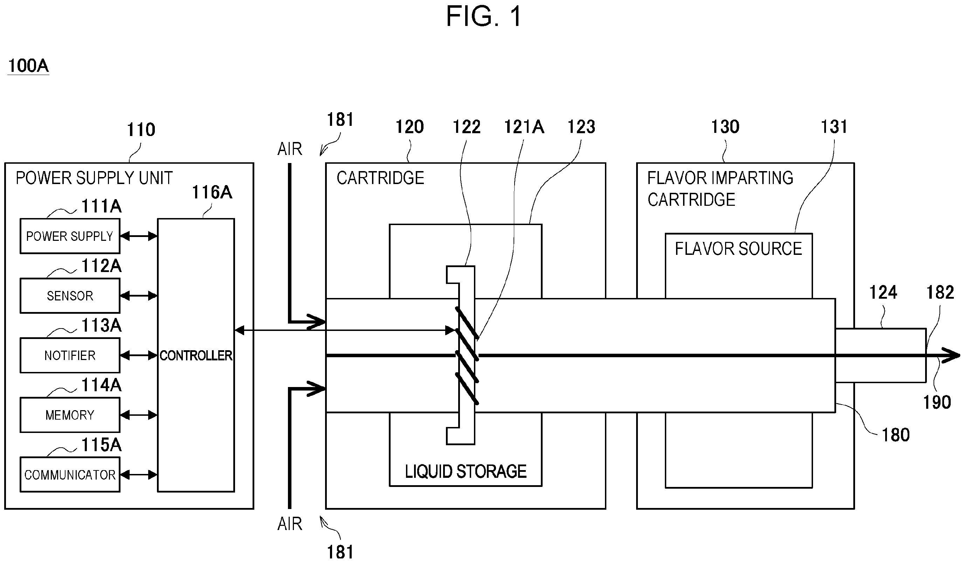

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a microphone condenser, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark), Bluetooth (registered trademark), or the like.

The controller 116A functions as an arithmetic processing unit and a control circuit, and controls the overall operations of the inhaler device 100A in accordance with various programs. The controller 116A includes an electronic circuit such as a CPU (central processing unit) or a microprocessor, for example.

The liquid storage 123 stores an aerosol source. The aerosol source is atomized to generate an aerosol. The aerosol source is a liquid such as polyhydric alcohol or water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

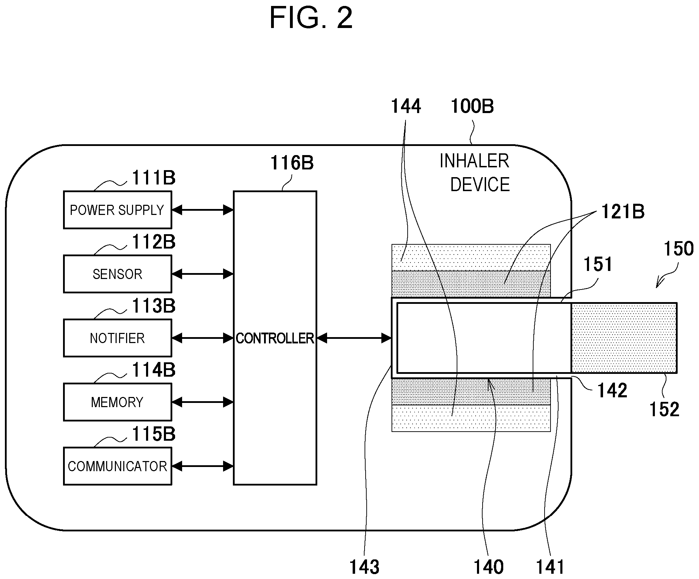

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining a path of air supplied to the stick substrate 150. For example, the bottom 143 has an air inlet hole that is an inlet of air into the airflow path. The opening 142, on the other hand, has an air outlet hole that is an outlet of the air from the airflow path.

The stick substrate 150 includes a substrate 151 and an inhalation port 152. The substrate 151 includes an aerosol source. The aerosol source according to the present configuration example is not limited to a liquid, and may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 from the airflow path (not illustrated), and the air and an aerosol generated from the substrate 151 reach inside the mouth of the user.

The heater 121B has the same configuration as the heater 121A according to the first configuration example. In the example illustrated in Fig. 2, however, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may accommodate the stick substrate 150 while sandwiching the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as an air inlet hole for the internal space 141. In this case, a mixture fluid of the air and an aerosol generated by the heater 121A flows into the internal space 141, mixes further with an aerosol generated by the heater 121B, and then reaches the oral cavity of the user

### «2. First Embodiment»

### (1) Configuration Example of System

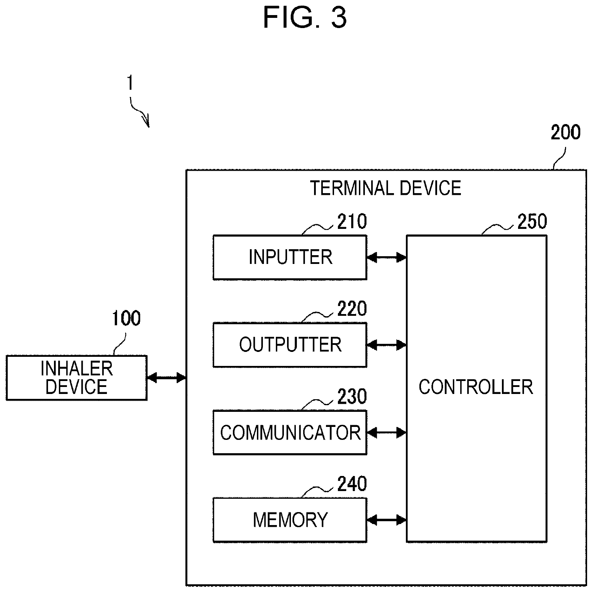

Fig. 3 is a diagram illustrating an example of the configuration of a system 1 according to the present embodiment. As illustrated in Fig. 3, the system 1 includes an inhaler device 100 and a terminal device 200.

### - Configuration of Inhaler Device 100

The inhaler device 100 according to the present embodiment generates, using a substrate, an aerosol to be inhaled by the user. A heater 121 is an example of a generation unit that generates an aerosol using a substrate. The cartridge 120 and the flavor imparting cartridge 130 in the first configuration example and the stick substrate 150 in the second configuration example are examples of the substrate in the present embodiment. The inhaler device 100 generates the aerosol using a substrate attached to the inhaler device 100. In the first configuration example, the cartridge 120 and the flavor imparting cartridge 130 connected to the power supply unit 110 are examples of the substrate attached to the inhaler device 100. In the second configuration example, the stick substrate 150 inserted into the inhaler device 100 is an example of the substrate attached to the inhaler device 100.

The heater 121 may heat an aerosol source as liquid guided from the substrate, instead. As described in the first configuration example, for example, the inhaler device 100 may generate the aerosol by heating the aerosol source guided by the liquid guide 122 from the liquid storage 123.

The heater 121 may heat a substrate containing an aerosol source and formed in a certain shape, instead. An example of the certain shape is a stick. As described in the second configuration example, for example, the inhaler device 100 may generate the aerosol by heating the stick substrate 150. Another example of the certain shape is a card. Another example of the certain shape is a cube.

The inhaler device 100 can take any of the above-described first and second configuration examples. An example of a case where the inhaler device 100 takes the second configuration example will be mainly described hereinafter in order to simplify description. In addition, in the following description, inhalation, by the user, of the aerosol generated by the inhaler device 100 will be simply referred to as an "inhalation" or a "puff". In addition, when the user inhales, it will be paraphrased as the "user puffs" hereinafter.

### - Configuration of Terminal Device 200

The terminal device 200 is a device used by the user of the inhaler device 100. For example, the terminal device 200 is any information processing device such as a smartphone, a tablet terminal, or a wearable device. As illustrated in FIG. 3, the terminal device 200 includes an inputter 210, an outputter 220, a communicator 230, a memory 240, and a controller 250.

The inputter 210 has a function of receiving inputs of various pieces of information. The inputter 210 may include an input device for receiving inputs of information from the user. The input device is, for example, a button, a keyboard, a touch panel, a microphone, or the like. The inputter 210 may also include various sensors including an image sensor.

The outputter 220 has a function of outputting information. The outputter 220 may include an output device that outputs information for the user. The output device is, for example, a display device that displays information, a light emission device that emits light, a vibration device that vibrates, a sound output device that outputs sound, or the like. An example of the display device is a display. An example of the light emission device is an LED (light-emitting diode). An example of the vibration device is an eccentric motor. An example of the sound output device is a speaker. The outputter 220 notifies the user of information input from the controller 250 by outputting the information.

The communicator 230 is a communication interface for communicating information between the terminal device 200 and another device. The communicator 230 performs communication based on any wired or wireless communication standard. Such a communication standard may be, for example, USB (universal serial bus), Wi-Fi (registered trademark), Bluetooth (registered trademark), or the like.

The memory 240 stores various pieces of information. The memory 240 is, for example, a nonvolatile storage medium such as a flash memory.

The controller 250 functions as an arithmetic processing device or a control device and controls overall operations in the terminal device 200 in accordance with various programs. The controller 250 is achieved, for example, by an electronic circuit such as a CPU (central processing unit) or a microprocessor. The controller 250 may also include a ROM (read-only memory) that stores programs to be used and arithmetic parameters and a RAM (random-access memory) that temporarily stores parameters which change as necessary and the like. The terminal device 200 performs various types of processing under control of the controller 250. Processing of information input using the inputter 210, output of information using the outputter 220, communication of information performed by the communicator 230, and storing and reading of information performed by the memory 240 are examples of processing controlled by the controller 250. The controller 250 also controls other types of processing performed by the terminal device 200, such as input of information to each component and processing based on information output from each component.

The functions of the controller 250 may be achieved by applications. The applications may be preinstalled or downloaded. Alternatively, the functions of the controller 250 may be achieved by PWAs (progressive web apps).

### - Communication between Devices

The inhaler device 100 can communicate with other devices. A communication link used for the communication between the inhaler device 100 and other devices may be wireless or wired. The present embodiment will be described on an assumption that the communication link is wireless.

The inhaler device 100 establishes connections especially with other devices paired therewith and communicates information. In pairing, two devices exchange and save information regarding the two devices. Examples of the information to be exchanged include identification information regarding a paired device, such as SSIDs (service set identifiers) and information regarding encryption keys to be used to encrypt information to be communicated.

The inhaler device 100 and the terminal device 200 perform pairing first, and then communicate information. A wireless communication standard used for wireless communication between the inhaler device 100 and the terminal device 200 is desirably a short-distance wireless communication standard such as Bluetooth. In this case, the inhaler device 100 and the terminal device 200 can establish a connection and communicate with each other if located within a range where short-distance wireless communication is possible. It is assumed in the following description that the inhaler device 100 and the terminal device 200 perform communication based on BLE (Bluetooth Low Energy (registered trademark)).

### (2) Locking Function

The inhaler device 100 has a locking function. The locking function is a function of controlling whether the heater 121 is to produce heat. Inhibition of the heating performed by the heater 121 will also be referred to as locking hereinafter. Permission of the heating performed by the heater 121 will also be referred to as unlocking.

When a user operation for requesting a start of the heating is performed in an unlocked state, the inhaler device 100 starts the heating using the heater 121. An example of the operation for requesting a start of the heating is pressing of a button provided for the inhaler device 100. When a user operation for requesting a start of the heating is performed in a locked state, on the other hand, the inhaler device 100 does not start the heating performed by the heater 121. With this configuration, since the heating by the heater 121 does not start in the locked state should the button be pressed by mistake inside a bag, for example, safety in use of the inhaler device 100 can be improved.

If a first condition is satisfied, the inhaler device 100 unlocks. When a plurality of first conditions are set, the inhaler device 100 may unlock if one of the plurality of first conditions is satisfied.

The first condition may include detection of operations corresponding to a preset first operation pattern. An operation pattern refers to a combination of operations performed on operation units that are provided for the inhaler device 100 and that are capable of receiving physical operations. The inhaler device 100 may be provided with a button and a slider for opening and closing the opening 142, for example, as the operation units. In this case, a combination of pressing of the button and an operation for opening and closing the opening 142 is set as the first operation pattern. With this configuration, the inhaler device 100 does not unlock unless the operations corresponding to the first operation pattern are performed. As a result, misuse by a person other than the user, such as a child, can be prevented, and the safety in use of the inhaler device 100 can be improved.

The first condition may include communication that is being performed between the inhaler device 100 and the terminal device 200. Here, an example of the communication that is being performed between the inhaler device 100 and the terminal device 200 is establishment of a connection between the inhaler device 100 and the terminal device 200 based on a short-distance wireless communication standard such as BLE. With this configuration, the inhaler device 100 unlocks when the inhaler device 100 and the terminal device 200 are located within a range where short-distance wireless communication is possible, and does not unlock when the inhaler device 100 and the terminal device 200 are not located within the range. When the user is going out carrying the terminal device 200, therefore, misuse, by a child, for example, of the inhaler device 100 left at home can be prevented. As a result, the safety in use of the inhaler device 100 can be improved. When the user uses the inhaler device 100 while carrying the terminal device 200, on the other hand, the inhaler device 100 automatically unlocks, thereby sparing the user from performing an unlocking operation and increasing usability.

If a second condition is satisfied, the inhaler device 100 locks. When a plurality of second conditions are set, the inhaler device 100 may lock if one of the plurality of second conditions is satisfied.

The second condition may include detection of operations corresponding to a preset second operation pattern. With this configuration, the inhaler device 100 can be locked on the basis of explicit operations performed by the user.

The second condition may include an end of communication between the inhaler device 100 and the terminal device 200. That is, the second condition may include an end of a connection established between the inhaler device 100 and the terminal device 200 based on a short-distance wireless communication standard such as BLE. With this configuration, when the user is going out carrying the terminal device 200, the inhaler device 100 left at home automatically locks.

### (3) Calculation of Time at Which Log Information Was Obtained

The inhaler device 100 includes a counter. The counter is a logical or physical component for counting a number. The counter included in the inhaler device 100 will also be referred to as a first counter hereinafter.

The inhaler device 100 changes a value of the first counter each time unit time has elapsed. An example of the change is counting up. The first counter may count the number of clock pulses of the CPU included in the controller 116, for example, and in this case, the unit time corresponds to the length of each clock pulse. Alternatively, the first counter may be an RTC (real-time clock), and the unit time may be 1 second. The inhaler device 100 starts to change the value of the first counter when the inhaler device 100 is activated for a first time. With this configuration, it is guaranteed that the first counter indicates different values at different timings. Time, therefore, can be calculated on the basis of the value of the first counter.

The inhaler device 100 stores log information including the value of the first counter at a first timing, at which a certain operation relating to the inhaler device 100 was performed. The log information is information obtained in response to use of the inhaler device 100 by the user. For example, the inhaler device 100 stores identification information indicating the certain operation and the value of the first counter at a timing when the certain operation was performed while associating the identification information and the value with each other. With this configuration, the terminal device 200 can calculate, as described later, a time corresponding to the first timing at which the certain operation relating to the inhaler device 100 was performed.

The inhaler device 100 transmits the stored log information at a second timing and also transmits the value of the first counter at the second timing. As a result, the terminal device 200 receives the log information including the value of the first counter at the first timing, at which the certain operation relating to the inhaler device 100 was performed, and the value of the first counter at the second timing, at which the inhaler device 100 transmitted the log information. The terminal device 200 then calculates the time corresponding to the first timing on the basis of the value of the first counter at the first timing, the value of the first counter at the second timing, and a time at which the terminal device 200 received the log information. The time corresponding to the first timing here is a time indicated by a clock included in the terminal device 200 when the certain operation relating to the inhaler device 100 was performed. The time at which the terminal device 200 received the log information corresponds to the value of the first counter at the second timing. A time obtained by multiplying a difference between the value of the first counter at the first timing and the value of the first counter at the second timing by the unit time of the first counter corresponds to a period of time from the first timing to the second timing. The terminal device 200, therefore, calculates a time the calculated period of time from the first timing to the second timing before the time at which the terminal device 200 received the log information as the time corresponding to the first timing.

As an example, an example is assumed where the time at which the terminal device 200 received the log information was 10:15. It is assumed that the value of the first counter at the first timing was 400 and the value of the first counter at the second timing was 1000. It is assumed that the unit time of the first counter was 1 second. In this case, 600 seconds, which is obtained by multiplying 600, which is obtained by subtracting 400 from 1000, by 1 second, which is the unit time, is the period of time from the first timing to the second timing. 10:05, which is 600 seconds before 10:15, therefore, is calculated as the time corresponding to the first timing.

With this configuration, the terminal device 200 can calculate the time corresponding to the first timing. In addition, even when the inhaler device 100 does not include a clock, or even when there is a discrepancy between the clock included in the inhaler device 100 and the clock included in the terminal device 200, the time corresponding to the first timing based on the clock included in the terminal device 200 can be obtained.

The inhaler device 100 may transmit information indicating the unit time of the first counter. In this case, the terminal device 200 calculates the time corresponding to the first timing further on the basis of the unit time of the first counter indicated by the received information. More specifically, when calculating the period of time from the first timing to the second timing, the terminal device 200 multiplies the difference between the value of the first counter at the first timing and the value of the first counter at the second timing by the unit time of the first counter indicated by the received information. With this configuration, an accurate time can be calculated even when the unit time of the first counter is different between inhaler devices 100.

### (4) Example of Certain Operation

The certain operation may include the heater 121 starting to generate the aerosol. For example, the inhaler device 100 stores log information including the value of the first counter at a timing when a user operation for requesting a start of the heating was performed and the heater 121 started the heating. With this configuration, the terminal device 200 can calculate a time at which the inhaler device 100 started to generate the aerosol.

The certain operation may include inhalation of the aerosol by the user. For example, the inhaler device 100 stores log information including the value of the first counter at a timing when a value accompanying a puff by the user, such as a decrease in temperature of the heater 121 or an increase in an airflow rate of the airflow path 180. With this configuration, the terminal device 200 can calculate a time at which the user puffed.

The certain operation may include charging of a power supply 111 that accumulates power and that supplies the power for the operation of the inhaler device 100. When the inhaler device 100 is connected to an external power supply through USB or the like, for example, the inhaler device 100 starts to charge the power supply 111. In this case, the inhaler device 100 stores log information including the value of the first counter at a timing when the inhaler device 100 was connected to the external power supply and started the charging. With this configuration, the terminal device 200 can calculate a time at which the inhaler device 100 started the charging.

The certain operation may include an operation that satisfies the first condition set for the locking function. For example, the inhaler device 100 stores log information including the value of the first counter at a timing when the inhaler device 100 unlocked on the basis of detection of the operations corresponding to the preset first operation pattern or establishment of a connection between the inhaler device 100 and the terminal device 200. With this configuration, the terminal device 200 can calculate a time at which the inhaler device 100 unlocked.

The certain operation may include an operation that satisfies the second condition set for the locking function. For example, the inhaler device 100 stores log information including the value of the first counter at a timing when the inhaler device 100 locked on the basis of detection of the operations corresponding to the preset second operation pattern or an end of a connection between the inhaler device 100 and the terminal device 200. With this configuration, the terminal device 200 can calculate a time at which the inhaler device 100 locked.

### (5) Process after Inhaler Device 100 Transmits Log Information

The inhaler device 100 may remove transmitted log information. Alternatively, the inhaler device 100 may overwrite transmitted log information stored in a memory area with new log information. In any case, the memory area storing the transmitted log information can be used to store the new log information, and the memory 114 can be efficiently used.

After calculating the time corresponding to the first timing, the terminal device 200 may transmit an acknowledgment indicating that log information has been successfully received. After receiving the acknowledgment indicating that log information has been successfully received, the inhaler device 100 may remove the transmitted log information or overwrite the transmitted log information stored in a memory area with new log information. With this configuration, if the terminal device 200 fails to receive log information or calculate the time corresponding to the first timing, the inhaler device 100 can avoid removal or overwriting of log information. If the inhaler device 100 does not receive an acknowledgment indicating that log information has been successfully received, the inhaler device 100 may transmit the transmitted log information again. With this configuration, the terminal device 200 can repeat receiving log information and calculating the time corresponding to the first timing until the log information is successfully received and the time is successfully calculated.

### (6) Process after Terminal Device 200 Receives Log Information

The terminal device 200 outputs the time corresponding to the first timing. For example, the terminal device 200 may output information indicating a certain operation relating to the inhaler device 100 performed at the first timing along with the time corresponding to the first timing. With this configuration, the user can view their activity history including times at which the heating started, times at which the user puffed, times at which the inhaler device 100 unlocked, and times at which the inhaler device 100 locked, along with the time corresponding to the first timing.

The terminal device 200 may include, as the inputter 210, a positional information obtainer that obtains positional information regarding the terminal device 200. The positional information obtainer receives a GNSS (global navigation satellite system) signal from a GNSS satellite (e.g., a GPS (global positioning system) signal from a GPS satellite), for example, detects positional information including latitude and longitude thereof, and outputs the detected positional information. The terminal device 200 may store the positional information obtained by the positional information obtainer while associating the positional information with a time at which the positional information was obtained. The terminal device 200 may then output positional information obtained at the time corresponding to the first timing. With this configuration, the user can view their activity history including positions at which the heating started, positions at which the user puffed, positions at which the inhaler device 100 unlocked, and positions at which the inhaler device 100 locked, along with a position of the terminal device 200.

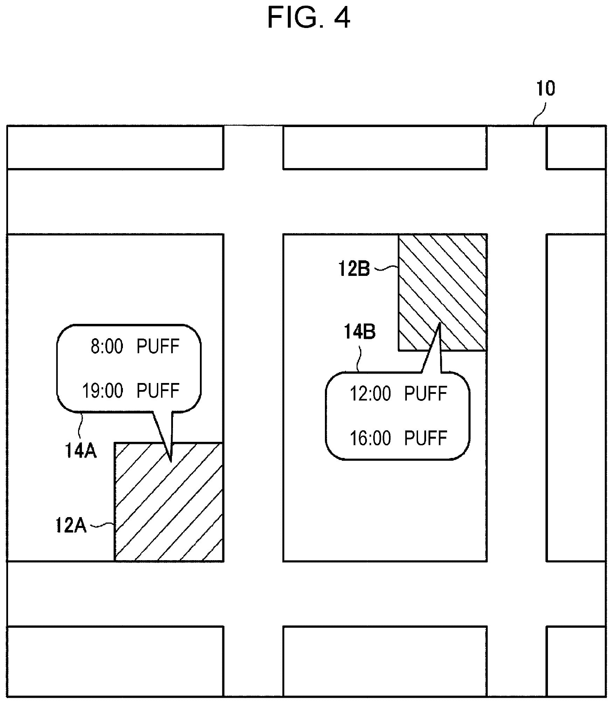

An example of log information output by the terminal device 200 will be described with reference to Fig. 4.

Fig. 4 is a diagram illustrating an example of a display screen for log information displayed by the terminal device 200 according to the present embodiment. In a display screen 10 illustrated in Fig. 4, the first timing is a timing at which the user puffed. In the display screen 10 illustrated in Fig. 4, positions 12A and 12B corresponding to positional information obtained at times when the user puffed are displayed on a map. For example, the position 12A indicates the user's home, and the position 12B indicates the user's workplace. Furthermore, in the display screen 10 illustrated in Fig. 4, the times at which the user puffed at the positions 12A and 12B are displayed. With this configuration, the user can easily recognize times and positions at which the user puffed.

The terminal device 200 may convert the value of the first counter included in the log information into a calculated time. The terminal device 200 may then transfer the log information after the conversion to another device. An example of the other device is a server that provides services relating to the inhaler device 100. The server may collect and analyze log information to use the log information for services including update of firmware of the inhaler device 100.

### (7) Procedure of Process

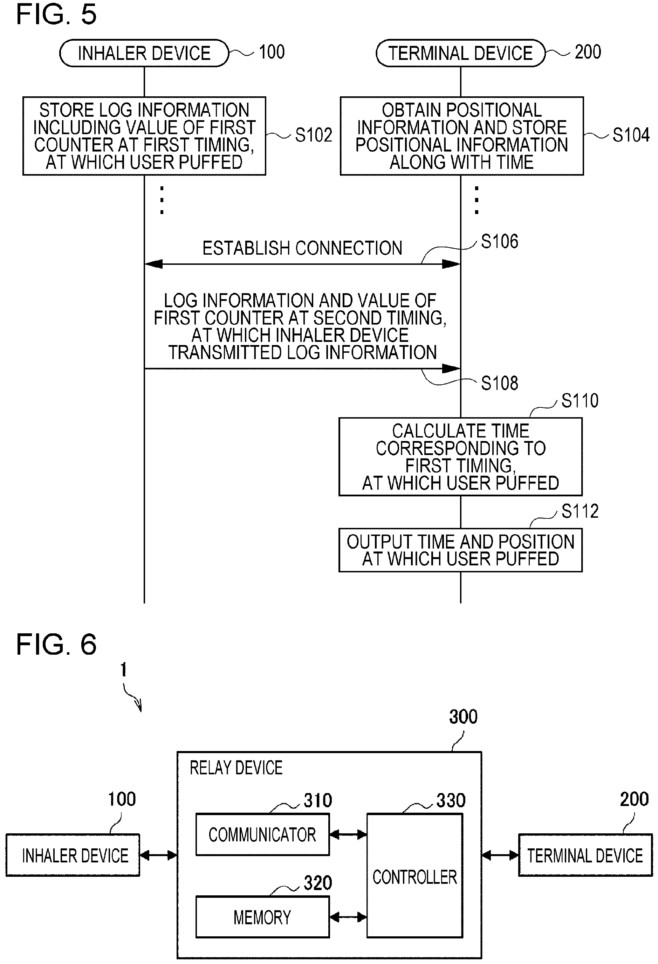

Fig. 5 is a sequence diagram illustrating an example of a procedure of a process performed by the system 1 according to the present embodiment. The inhaler device 100 and the terminal device 200 are involved in this sequence. A process when log information relating to a puff is obtained as an example of the certain operation relating the inhaler device 100 will be described hereinafter.

As illustrated in Fig. 5, first, when the user puffs, the inhaler device 100 stores log information including the value of the first counter at the first timing, at which the user puffed (step S102). The inhaler device 100 performs the processing in step S102 each time the user puffs.

The terminal device 200, on the other hand, obtains positional information and stores the positional information along with a time (step S104). The terminal device 200 repeatedly performs the processing in step S104 as time elapses.

Next, the inhaler device 100 and the terminal device 200 establish a connection (step S106). When the inhaler device 100 and the terminal device 200 are located within a range where short-distance wireless communication is possible and the certain user operation is performed on the inhaler device 100, for example, the inhaler device 100 and the terminal device 200 establish a connection.

Next, the inhaler device 100 transmits the log information stored in step S102 and also transmits the value of the first counter at the second timing, at which the inhaler device 100 transmitted the log information (step S108). The terminal device 200 receives these pieces of information.

The terminal device 200 then calculates a time corresponding to the first timing, at which the user puffed, on the basis of the received information and a time at which the terminal device 200 received the log information (step S110). More specifically, first, the terminal device 200 calculates a period of time from the first timing to the second timing by multiplying a difference between the value of the first counter at the first timing, at which the user puffed, and the value of the first counter at the second timing, at which the inhaler device 100 transmitted the log, by the unit time of the first counter. The terminal device 200 then calculates, as the time corresponding to the first timing, a time the calculated period of time before the time at which the terminal device 200 received the log information.

Next, the terminal device 200 outputs the time and a position at which the user puffed (step S112). More specifically, the terminal device 200 displays the time calculated in step S110 and the positional information obtained in step S104 at the time as information indicating the time and the position at which the user puffed. A specific example of the displayed information is as described with reference to Fig. 4.

### «3. Second Embodiment»

In the first embodiment, the inhaler device 100 and the terminal device 200 directly communicate with each other. In a second embodiment, in contrast, the inhaler device 100 and the terminal device 200 indirectly communicate with each other through another device.

### (1) Configuration Example of System

Fig. 6 is a diagram illustrating an example of the configuration of the system 1 according to the present embodiment. As illustrated in Fig. 6, the system 1 includes the inhaler device 100, the terminal device 200, and a relay device 300.

### - Configuration of Relay Device 300

The relay device 300 is a device that relays communication between the inhaler device 100 and the terminal device 200. For example, the relay device 300 is a charger that charges the inhaler device 100 when connected to the inhaler device 100. As illustrated in Fig. 6, the relay device 300 includes a communicator 310, a memory 320, and a controller 330.

The communicator 310 is a communication interface for communicating information between the relay device 300 and another device. The communicator 310 performs communication based on any wired or wireless communication standard. Such a communication standard may be, for example, USB (universal serial bus), Wi-Fi (registered trademark), Bluetooth (registered trademark), or the like.

The memory 320 stores various pieces of information. The memory 320 is, for example, a nonvolatile storage medium such as a flash memory.

The controller 330 functions as an arithmetic processing device or a control device and controls overall operations of the relay device 300 in accordance with various programs. The controller 330 is, for example, an electronic circuit such as a CPU (central processing unit) or a microprocessor. Alternatively, the controller 330 may include a ROM (read-only memory) that stores programs to be used, arithmetic parameters, and the like and a RAM (random-access memory) that temporarily stores parameters that change as necessary and the like. The relay device 300 performs various types of processing under control of the controller 330. The communication of information by the communicator 310 and the storing and reading of information by the memory 320 are examples of processing controlled by the controller 330. The controller 330 also controls other types of processing performed by the relay device 300, such as input of information to each component and processing based on information output from each component.

### - Configuration of Inhaler Device 100

The inhaler device 100 communicates with the terminal device 200 through the relay device 300. For example, the terminal device 200 does not have a wireless communication function but has a wired communication function, and communicates with the relay device 300 when connected to the relay device 300. Other parts of the configuration are the same as in the first embodiment.

### - Configuration of Terminal Device 200

The terminal device 200 communicates with the inhaler device 100 through the relay device 300. Other parts of the configuration are the same as in the first embodiment.

### (2) First Example of Calculation of Time Corresponding to First Timing

The relay device 300 can simultaneously communicate with the inhaler device 100 and the terminal device 200. At the second timing, the relay device 300 relays log information received from the inhaler device 100 to the terminal device 200. That is, the relay device 300 immediately (i.e., without delay) transfers the log information received from the inhaler device 100 to the terminal device 200. The relay device 300 also relays the value of the first counter at the second timing to the terminal device 200. In this case, there is only a negligible, if not no, time lag between the time at which the inhaler device 100 transmitted the log information and the time at which the terminal device 200 received the log information.

The terminal device 200, therefore, can calculate the time corresponding to the first timing using the same method as in the first embodiment. That is, the terminal device 200 calculates the time corresponding to the first timing on the basis of the value of the first counter at the first timing, the value of the first counter at the second timing, and the time at which the terminal device 200 received the log information. More specifically, the terminal device 200 calculates, as the time corresponding to the first timing, a time a period of time from the first timing to the second timing, which is calculated on the basis of a difference between two values of the first counter, before the time at which the terminal device 200 received the log information. With this configuration, even when the relay device 300 relays the communication between the inhaler device 100 and the terminal device 200, the terminal device 200 can calculate the time corresponding to the first timing.

### (3) Second Example of Calculation of Time Corresponding to First Timing

The relay device 300 can communicate with the inhaler device 100 and the terminal device 200 at different timings. For example, the relay device 300 stores information received from the inhaler device 100 when the relay device 300 communicates with the inhaler device 100. The relay device 300 then transmits the information received from the inhaler device 100 to the terminal device 200 when communicating with the terminal device 200. In this case, a large time lag that cannot be ignored is caused between the time at which the inhaler device 100 transmits the log information and the time at which the terminal device 200 receives the log information. A method for calculating the time corresponding to the first timing will be described hereinafter in consideration of such a time lag.

The relay device 300 includes a counter. The counter included in the relay device 300 will also be referred to as a second counter hereinafter.

The relay device 300 changes a value of the second counter each time the unit time has elapsed. An example of the change is counting up. The second counter may count the number of clock pulses of the CPU included in the controller 330, for example, and in this case, the unit time corresponds to the length of each clock pulse. Alternatively, the second counter may be an RTC (real-time clock), and the unit time may be 1 second. The relay device 300 starts to change the value of the second counter when the relay device 300 is activated for a first time. With this configuration, it is guaranteed that the second counter indicates different values at different timings. Time, therefore, can be calculated on the basis of the value of the second counter.

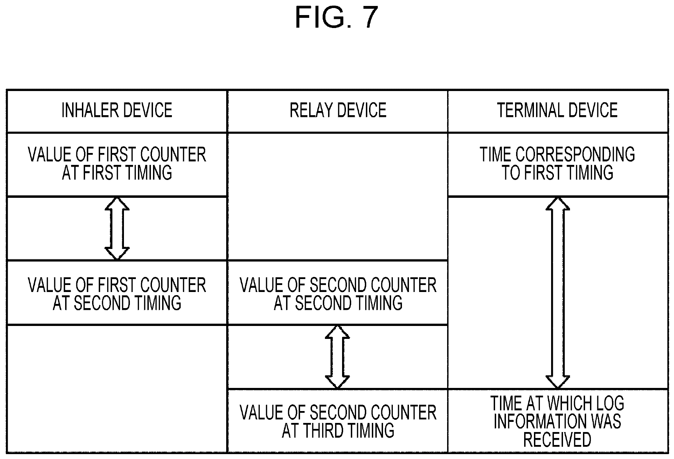

The method for calculating the time corresponding to the first timing will be described hereinafter with reference to Fig. 7. Fig. 7 is a diagram illustrating the method for calculating the time corresponding to the first timing according to the present embodiment.

As described above in the first embodiment, the inhaler device 100 stores log information including the value of the first counter at the first timing. The inhaler device 100 transmits the log information at the second timing and also transmits the value of the first counter at the second timing. As illustrated in Fig. 7, the period of time between the first timing and the second timing corresponds to the difference between the value of the first counter at the first timing and the value of the first counter at the second timing.

The relay device 300 receives the log information from the inhaler device 100 at the second timing and then receives the value of the first counter at the second timing from the inhaler device 100. The relay device 300 then stores the value of the second counter at the second timing along with the log information and the value of the first counter at the second timing received from the inhaler device 100. As illustrated in Fig. 7, the value of the first counter at the second timing corresponds to the value of the second counter at the second timing.

Thereafter, the relay device 300 transmits the log information at a third timing, at which the relay device 300 communicates with the terminal device 200, and also transmits the value of the first counter at the second timing, the value of the second counter at the second timing, and the value of the second counter at the third timing. As illustrated in Fig. 7, a period of time between the second timing and the third timing corresponds to a difference between the value of the second counter at the second timing and the value of the second counter at the third timing.

The terminal device 200 then calculates the time corresponding to the first timing on the basis of the value of the first counter at the first timing, the value of the first counter at the second timing, the value of the second counter at the second timing, the value of the second counter at the third timing, and the time at which the terminal device 200 received the log information. The time corresponding to the first timing is a time indicated by the clock included in the terminal device 200 when the certain operation relating to the inhaler device 100 was performed. As illustrated in Fig. 7, the time at which the terminal device 200 received the log information corresponds to the value of the second counter at the third timing. A period of time obtained by multiplying a difference between the value of the second counter at the second timing and the value of the second counter at the third timing by the unit time of the second counter corresponds to a period of time from the second timing to the third timing. A period of time obtained by multiplying a difference between the value of the first counter at the first timing and the value of the first counter at the second timing by the unit time of the first counter corresponds to the period of time from the first timing to the second timing. The terminal device 200 then calculates, as the time corresponding to the first timing, a time the sum of the calculated period of time from the second timing to the third timing and the calculated period of time from the first timing to the second timing before the time at which the terminal device 200 received the log information.

As an example, an example is assumed where the time at which the terminal device 200 received the log information was 10:25. It is assumed that the value of the first counter at the first timing was 400 and the value of the first counter at the second timing was 1000. It is assumed that the value of the second counter at the second timing was 1400 and the value of the second counter at the third timing was 2000. It is assumed that the unit time of the first counter and the unit time of the second counter were 1 second. In this case, 600 seconds, which is obtained by multiplying 600, which is obtained by subtracting 1400 from 2000, by 1 second, which is the unit time of the second counter, is the period of time from the second timing to the third timing. 10:15, which is 600 seconds before 10:25, therefore, is calculated as the time corresponding to the second timing. In addition, 600 seconds, which is obtained by multiplying 600, which is obtained by subtracting 400 from 1000, by 1 second, which is the unit time of the first counter, is the period of time from the first timing to the second timing. 10:05, which is 600 seconds before 10: 15, which corresponds to the second timing, therefore, is calculated as the time corresponding to the first timing.

As described above, even if a large time lag that cannot be ignored is caused between the time at which the inhaler device 100 transmitted the log information and the time at which the terminal device 200 received the log information, the terminal device 200 can calculate the time corresponding to the first timing.

The unit time of the first counter and the unit time of the second counter may be different from each other. The relay device 300 may transmit information indicating the unit time of the second counter to the terminal device 200. In addition, the relay device 300 may transfer, to the terminal device 200, information indicating the first unit time transmitted from the inhaler device 100. In this case, the terminal device 200 calculates the time corresponding to the first timing further on the basis of the unit time of the first counter and the unit time of the second counter indicated by the received information. With this configuration, even when the unit time of the first counter is different between inhaler devices 100 and the unit time of the second counter is different between relay devices 300, an accurate time can be calculated.

### «4. Appendix»

Although preferred embodiments of the present invention have been described in detail above with reference to the accompanying drawings, the present invention is not limited to these examples. It is obvious that those who have ordinary knowledge in the technical field to which the present invention belongs can come up with various modifications or corrections within the scope of the technical idea described in the claims, and it should be understood that these modifications or corrections naturally belong to the technical scope of the present disclosure.

The processes performed by the devices described herein may be achieved by software, hardware, or a combination of software and hardware. Programs constituting software are stored in advance in storage media (non-transitory media) provided inside or outside the devices. When each of the programs is executed, for example, a computer that controls a corresponding one of the devices described herein loads the program into a RAM, and a processor such as a CPU executes the program. The storage media may include, for example, a magnetic disk, an optical disc, a magneto-optical disk, and a flash memory. The computer programs may be distributed over a network, for example, without using the storage media.

The processes described using flowcharts and sequence diagrams herein need not necessarily be performed in illustrated order. Some processing steps may be performed in parallel with each other. Additional processing steps may be employed, and some processing steps may be omitted.

The following configurations also belong to the technical scope of the present invention.
(1) An inhaler device including:
   a generation unit that generates an aerosol using a substrate;
   a memory that stores information; and
   a controller that controls operation of the inhaler device,
   in which the controller changes a value of a first counter each time unit time has elapsed, and
   in which the memory stores log information including the value of the first counter at a first timing, at which a certain operation relating to the inhaler device was performed.
(2) The inhaler device according to (1),
   in which the certain operation includes the generation unit starting to generate the aerosol.
(3) The inhaler device according to (1) or (2),
   in which the certain operation includes inhalation of the aerosol by the user.
(4) The inhaler device according to any of (1) to (3), further including:
   a power supply that accumulates power and that supplies the power for operation of the inhaler device,
   in which the certain operation includes charging of the power supply.
(5) The inhaler device according to any of (1) to (4),
   in which, if a first condition is satisfied, the controller permits the generation of the aerosol, and
   in which the certain operation includes an operation that satisfies the first condition.
(6) The inhaler device according to any of (1) to (5),
   in which, if a second condition is satisfied, the inhaler device inhibits the generation of the aerosol, and
   in which the certain operation includes an operation that satisfies the second condition.
(7) The inhaler device according to any of (1) to (6), further including:
   a communicator that communicates with another device,
   in which the communicator transmits the log information stored in the memory at a second timing and also transmits the value of the first counter at the second timing.
(8) The inhaler device according to (7),
   in which the communicator transmits information indicating the unit time.
(9) The inhaler device according to (7) or (8),
   in which the memory removes the transmitted log information or overwrites the transmitted log information stored in a memory area with the new log information.
(10) The inhaler device according to (7) or (8),
   in which, after the communicator receives an acknowledgment indicating that the log information was successfully received, the memory removes the transmitted log information or overwrites the transmitted log information stored in a memory area with new log information.
(11) The inhaler device according to any of (1) to (10),
   in which the controller starts to change the value of the first counter when the inhaler device is activated for a first time.
(12) The inhaler device according to any of (1) to (11),
   in which the generation unit heats an aerosol source as liquid guided from the substrate.
(13) The inhaler device according to any of (1) to (12),
   in which the generation unit heats the substrate that contains an aerosol source and that is formed in a certain shape.
(14) A terminal device including:
   a communicator that communicates with another device; and
   a controller that performs processing based on information received by the communicator,
   in which the communicator receives log information including a value of a first counter at a first timing, at which a certain operation relating to an inhaler device, which generates an aerosol using a substrate, changes the value of the first counter each time unit time has elapsed, and stores the log information, was performed, and the value of the first counter at a second timing, at which the inhaler device transmitted the log information, and
   in which the controller calculates a time corresponding to the first timing on a basis of the value of the first counter at the first timing, the value of the first counter at the second timing, and a time at which the communicator received the log information.
(15) The terminal device according to (14),
   in which, after the time corresponding to the first timing is calculated, the communicator transmits an acknowledgment indicating that the log information was successfully received.
(16) The terminal device according to (14) or (15), further including:
   an outputter that outputs information,
   in which the outputter outputs the time corresponding to the first timing.
(17) The terminal device according to (16), further including:
   a positional information obtainer that obtains positional information regarding the terminal device; and
   a memory that stores the positional information obtained by the positional information obtainer while associating the positional information with a time at which the positional information was obtained,
   in which the outputter outputs the positional information obtained at the time corresponding to the first timing.
(18) The terminal device according to any of (14) to (17),
   in which the terminal device communicates with the inhaler device through a relay device that communicates with the inhaler device,
   in which the relay device relays, at the second timing, the log information received from the inhaler device to the relay device and also relays the value of the first counter at the second timing to the terminal device, and
   in which the controller calculates the time corresponding to the first timing on a basis of the value of the first counter at the first timing, the value of the first counter at the second timing, and the time at which the communicator received the log information.
(19) The terminal device according to any of (14) to (17),
   in which the communicator communicates with the inhaler device through a relay device that communicates with the inhaler device,
   in which the relay device
   changes a value of a second counter each time unit time has elapsed,
   receives the log information from the inhaler device at the second timing and then receives the value of the first counter at the second timing from the inhaler device,
   stores the value of the second counter at the second timing along with the log information and the value of the first counter at the second timing, and
   transmits the log information at a third timing, at which the relay device communicates with the terminal device, and also transmits the value of the first counter at the second timing, the value of the second counter at the second timing, and the value of the second counter at the third timing, and
   in which the controller calculates the time corresponding to the first timing on a basis of the value of the first counter at the first timing, the value of the first counter at the second timing, the value of the second counter at the second timing, the value of the second counter at the third timing, and the time at which the communicator received the log information.
(20) A program for causing a computer, which controls a terminal device including a communicator that communicates with another device, to calculate, when an inhaler device, which generates an aerosol using a substrate, changes a value of a first counter each time unit time has elapsed, and stores log information, receives the log information including the value of the first counter at a first timing, at which a certain operation relating to the inhaler device was performed, and the value of the first counter at a second timing, at which the inhaler device transmitted the log information, a time corresponding to the first timing on a basis of the value of the first counter at the first timing, the value of the first counter at the second timing, and a time at which the communicator received the log information.

### Reference Signs List

- 1: system
- 100: inhaler device
- 110: power supply unit
- 111: power supply
- 112: sensor
- 113: notifier
- 114: memory
- 115: communicator
- 116: controller
- 120: cartridge
- 121: heater
- 122: liquid guide
- 123: liquid storage
- 124: mouthpiece
- 130: flavor imparting cartridge
- 131: flavor source
- 140: holder
- 141: internal space
- 142: opening
- 143: bottom
- 144: heat insulator
- 150: stick substrate
- 151: substrate
- 152: inhalation port
- 180: airflow path
- 181: air inlet hole
- 182: air outlet hole
- 200: terminal device
- 210: inputter
- 220: outputter
- 230: communicator
- 240: memory
- 250: controller
- 300: relay device
- 310: communicator
- 320: memory
- 330: controller

## Claims

1. An inhaler device comprising:
a generation unit that generates an aerosol using a substrate;
a memory that stores information; and
a controller that controls operation of the inhaler device,
wherein the controller changes a value of a first counter each time unit time has elapsed, and
wherein the memory stores log information including the value of the first counter at a first timing, at which a certain operation relating to the inhaler device was performed.

2. The inhaler device according to claim 1,
wherein the certain operation includes the generation unit starting to generate the aerosol.

3. The inhaler device according to claim 1 or 2,
wherein the certain operation includes inhalation of the aerosol by the user.

4. The inhaler device according to any of claims 1 to 3, further comprising:
a power supply that accumulates power and that supplies the power for operation of the inhaler device,
wherein the certain operation includes charging of the power supply.

5. The inhaler device according to any of claims 1 to 4,
wherein, if a first condition is satisfied, the inhaler device permits the generation of the aerosol, and
wherein the certain operation includes an operation that satisfies the first condition.

6. The inhaler device according to any of claims 1 to 5,
wherein, if a second condition is satisfied, the inhaler device inhibits the generation of the aerosol, and
wherein the certain operation includes an operation that satisfies the second condition.

7. The inhaler device according to any of claims 1 to 6, further comprising:
a communicator that communicates with another device,
wherein the communicator transmits the log information stored in the memory at a second timing and also transmits the value of the first counter at the second timing.

8. The inhaler device according to claim 7,
wherein the communicator transmits information indicating the unit time.

9. The inhaler device according to claim 7 or 8,
wherein the memory removes the transmitted log information or overwrites the transmitted log information stored in a memory area with the new log information.

10. The inhaler device according to claim 7 or 8,
wherein, after the communicator receives an acknowledgment indicating that the log information was successfully received, the memory removes the transmitted log information or overwrites the transmitted log information stored in a memory area with new log information.

11. The inhaler device according to any of claims 1 to 10,
wherein the controller starts to change the value of the first counter when the inhaler device is activated for a first time.

12. The inhaler device according to any of claims 1 to 11,
wherein the generation unit heats an aerosol source as liquid guided from the substrate.

13. The inhaler device according to any of claims 1 to 12,
wherein the generation unit heats the substrate that contains an aerosol source and that is formed in a certain shape.

14. A terminal device comprising:
a communicator that communicates with another device; and
a controller that performs processing based on information received by the communicator,
wherein the communicator receives log information including a value of a first counter at a first timing, at which a certain operation relating to an inhaler device, which generates an aerosol using a substrate, changes the value of the first counter each time unit time has elapsed, and stores the log information, was performed, and the value of the first counter at a second timing, at which the inhaler device transmitted the log information, and
wherein the controller calculates a time corresponding to the first timing on a basis of the value of the first counter at the first timing, the value of the first counter at the second timing, and a time at which the communicator received the log information.

15. The terminal device according to claim 14,
wherein, after the time corresponding to the first timing is calculated, the communicator transmits an acknowledgment indicating that the log information was successfully received.

16. The terminal device according to claim 14 or 15, further comprising:
an outputter that outputs information,
wherein the outputter outputs the time corresponding to the first timing.

17. The terminal device according to claim 16, further comprising:
a positional information obtainer that obtains positional information regarding the terminal device; and
a memory that stores the positional information obtained by the positional information obtainer while associating the positional information with a time at which the positional information was obtained,
wherein the outputter outputs the positional information obtained at the time corresponding to the first timing.

18. The terminal device according to any of claims 14 to 17,
wherein the terminal device communicates with the inhaler device through a relay device that communicates with the inhaler device,
wherein the relay device relays, at the second timing, the log information received from the inhaler device to the relay device and also relays the value of the first counter at the second timing to the terminal device, and
wherein the controller calculates the time corresponding to the first timing on a basis of the value of the first counter at the first timing, the value of the first counter at the second timing, and the time at which the communicator received the log information.

19. The terminal device according to any of claims 14 to 17,
wherein the communicator communicates with the inhaler device through a relay device that communicates with the inhaler device,
wherein the relay device
changes a value of a second counter each time unit time has elapsed,
receives the log information from the inhaler device at the second timing and then receives the value of the first counter at the second timing from the inhaler device,
stores the value of the second counter at the second timing along with the log information and the value of the first counter at the second timing, and
transmits the log information at a third timing, at which the relay device communicates with the terminal device, and also transmits the value of the first counter at the second timing, the value of the second counter at the second timing, and the value of the second counter at the third timing, and
wherein the controller calculates the time corresponding to the first timing on a basis of the value of the first counter at the first timing, the value of the first counter at the second timing, the value of the second counter at the second timing, the value of the second counter at the third timing, and the time at which the communicator received the log information.

20. A program for causing a computer, which controls a terminal device including a communicator that communicates with another device, to calculate, when an inhaler device, which generates an aerosol using a substrate, changes a value of a first counter each time unit time has elapsed, and stores log information, receives the log information including the value of the first counter at a first timing, at which a certain operation relating to the inhaler device was performed, and the value of the first counter at a second timing, at which the inhaler device transmitted the log information, a time corresponding to the first timing on a basis of the value of the first counter at the first timing, the value of the first counter at the second timing, and a time at which the communicator received the log information.
